(19) Europäisches Patentamt
European Patent Office
Office européen des brevets

(11) **EP 4 299 742 A1**

(12) # EUROPEAN PATENT APPLICATION

(43) Date of publication:
**03.01.2024 Bulletin 2024/01**

(21) Application number: **22305950.2**

(22) Date of filing: **30.06.2022**

(51) International Patent Classification (IPC):
**C12N 15/113** *(2010.01)* **A61K 31/7125** *(2006.01)*
**A61K 31/713** *(2006.01)* **A61K 31/712** *(2006.01)*
**A61P 35/00** *(2006.01)*

(52) Cooperative Patent Classification (CPC):
**C12N 15/113;** C12N 2310/113; C12N 2310/14;
C12N 2310/20; C12N 2310/315; C12N 2310/3231;
C12N 2310/341; C12N 2320/31

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
- **Université de Strasbourg
  67000 Strasbourg (FR)**
- **Institut National de la Santé et de la
  Recherche Médicale (INSERM)
  75013 Paris (FR)**
- **Centre national de la recherche scientifique
  75016 Paris (FR)**
- **Katholieke Universiteit Leuven
  3000 Leuven (BE)**

(72) Inventors:
- **DAVIDSON, Irwin
  67000 STRASBOURG (FR)**
- **GAMBI, Giovanni
  67000 STRASBOURG (FR)**
- **LEUCCI, Eleonora
  30000 LOUVAIN (BE)**

(74) Representative: **Cabinet Becker et Associés
25, rue Louis le Grand
75002 Paris (FR)**

Remarks:
The complete document including Reference
Table(s) and the Sequence Listing(s) can be
downloaded from the EPO website

(54) **COMBINATION THERAPY FOR MELANOMA**

(57) The present invention relates to the field of oncology. More particularly, the present invention relates to a new combination therapy for use in treating melanoma.

EP 4 299 742 A1

## Description

### INTRODUCTION

The present invention relates to the field of oncology. More particularly, the present invention relates to a new combination therapy for use in treating melanoma.

Melanoma, a highly aggressive cancer that develops from pigment-producing cells known as melanocytes, is the most dangerous and deadly type of skin cancer. It has become a major public health concern due to its incidence steadily rising over recent decades.

Melanoma patients are typically treated either by surgery at early stages, or by a combination of surgery, chemotherapy, targeted therapy and/or immunotherapy once the cancer has disseminated. Yet, the therapeutic rate is not satisfactory across patients, especially for those with distant metastasis. While detection of the disease in early stage may be curable (in the USA, the five-year survival rate after treatment is 99% for localized disease), later-stage disease that has spread has a relatively poor prognosis, with a median survival of less than 10 months for the most advanced melanoma (in the USA, the five-year survival rate after treatment is 65% when spread to lymph nodes, and only 25% with distant spread). Even if targeted therapy and immunotherapy have improved the overall survival of melanoma patients, some patients still fail to respond or become rapidly resistant to therapy.

There is thus still a need for new therapeutic options and combinations therapies for treating melanoma that act independently of current therapies, so as to notably increase the pool of concerned patients.

Without being bound by theory, the lack of response or emerging resistance to treatment of melanoma is thought to be due to the highly dynamic nature and intra-tumor heterogeneity of this cancer. Melanoma tumors are indeed notoriously heterogeneous comprising cell populations with distinct properties and gene expression signatures. For example, BRAF and NRAS mutations, which activate the mitogen-activated protein kinase (MAPK) pathway, are frequently observed mutations in melanoma patients, yet therapies targeting these particular mutations have either proved to be toxic and/or leading to a relapse after rapid tumor regression.

Molecular characterisation of melanoma subpopulations is therefore needed to facilitate the design of more sophisticated combinatorial approaches to reduce heterogeneity and improve therapeutic response.

Although rare, some vulnerabilities common to most melanoma cell states have been identified and successfully exploited to overcome therapy resistance, with a clear example being inhibition of mitochondrial function that impacts viability of multiple melanoma states irrespective of driver mutations leading to long lasting antitumor response.

Long non-coding (Lnc)RNAs, a form of non-coding RNA over 200 nucleotides in length that have low protein-coding potential, have recently emerged as important regulators of virtually every process in the cell, in particular adaptive processes involved in tumor progression and therapy resistance. Among these lncRNAs, LINC00518 (also known as LENOX) and LINC01212 (also known as SAMMSON) have been identified as weakly expressed in healthy melanocytes yet highly upregulated in all known melanoma states (but not in other human tumors); their use as suitable diagnostic markers for melanoma and/or as suitable targets for treatment of melanoma, irrespective of phenotype or mutation status, has been reported (WO2015024986; Leucci et al., Nature, 2016: 531:518-22; WO2021/152005).

The present Inventors are herein the first to investigate an innovative therapeutic approach aimed at targeting the two melanoma-specific lncRNAs (i) LINC00518 and (ii) SAMMSON, by knocking-down their respective functional expression. To do so, they assessed the efficacy of this proof-of-concept in melanoma cells that are either melanocytic or undifferentiated (mesenchymal), with different mutational status (e.g. BRAF or RNAS), using an antisense targeting LINC00518 in combination with an antisense targeting SAMMSON. Testing these two types of cells is important since those that are melanocytic are typically involved in the proliferative aspect of melanoma, while those of mesenchymal phenotype are generally responsible for the invasion and resistance to therapy. Melanoma cells resistant to BRAF inhibitors were also tested.

This combination therapy unexpectedly produced a synergistic improvement, thereby confirming the suitability of this approach for treating melanoma: indeed, the co-inhibition of LINC00518 and SAMMSON cooperates to induce the apoptosis and reduce the proliferation of melanoma cells, irrespective of their phenotype or mutational status (e.g. BRAF or RNAS), or resistance to conventional therapy. These results are all the more surprising given that such effects were obtained with relatively low doses of each antisense. It should nevertheless be understood that the combination of (i) any inhibitor of the functional expression of LINC00518 with (ii) any inhibitor of the functional expression of SAMMSON, would achieve the same effect.

### SUMMARY OF THE INVENTION

In a first aspect, the invention relates to an inhibitor of functional expression of the long-non coding RNA (lncRNA) LINC00518 for use in combination with (ii) an inhibitor of functional expression of the long-non coding RNA (lncRNA) SAMMSON, in the treatment of a melanoma.

In a preferred embodiment, the inhibitors (each and/or in combination) increase the apoptosis and/or decrease the proliferation of melanoma cells.

In a preferred embodiment, the inhibitors (each and/or in combination) increase apoptosis and/or decrease the proliferation of melanoma cells, independent of BRAF and/or NRAS status.

In a preferred embodiment, the melanoma is an advanced melanoma or a metastatic melanoma.

In a preferred embodiment, the melanoma is a resistant melanoma, in particular a melanoma resistant to chemotherapy, targeted therapy, and/or immune checkpoint inhibitors.

In a preferred embodiment, each inhibitor is either a nucleic acid molecule interfering specifically with the expression of the long-non coding RNA, or is a genome editing system comprising a nuclease engineered to target specifically the long-non coding RNA.

In a preferred embodiment, the nucleic acid molecule interfering specifically with the expression of the long-non coding RNA is an antisense nucleic acid, an RNAi nucleic acid, or a ribozyme, preferably is an antisense nucleic acid.

In a preferred embodiment, the antisense nucleic interfering specifically with the expression of the long-non coding RNA induces a RNAse H mediated degradation.

In a preferred embodiment, the antisense nucleic acid interfering specifically with the expression of the long-non coding RNA is a gapmer.

In a preferred embodiment, the antisense is an LNA antisense and/or comprises phosphothiorate linkages, preferably the gapmer is an LNA gapmer and/or comprises phosphothiorate linkages.

In a preferred embodiment, the genome editing system comprising a nuclease engineered to target specifically the long-non coding RNA is the CRISPR/Cas system, the Zinc-finger nuclease (ZFN) system, the TALEN system, or the meganuclease system, preferably is the CRISPR/Cas system such as the CRISPR/Cas9 system.

In a further aspect, the invention relates to (i) an inhibitor of functional expression of the long-non coding RNA (lncRNA) LINC00518 and (ii) an inhibitor of functional expression of the long-non coding RNA (lncRNA) SAMMSON, as described herein, as a combined preparation for simultaneous, separate or sequential use in the treatment of a melanoma.

In a further aspect, the invention relates to a pharmaceutical composition comprising (i) an inhibitor of functional expression of the long-non coding RNA (lncRNA) LINC00518 and (ii) an inhibitor of functional expression of the long-non coding RNA (lncRNA) SAMMSON, as described herein, and optionally (iii) a pharmaceutically acceptable excipient.

In a further aspect, the invention relates to an *in vitro* method for identifying a melanoma tumor suitable for treatment with the inhibitors of the invention, said method comprising:

a) determining the respective expression level of the long-non coding RNAs (lncRNAs) LINC00518 and SAMMSON, in a melanoma tumor sample;
b) comparing the expression level determined in step a) with a reference expression level for each of said lncRNA, thereby identifying whether the melanoma tumor is suitable for said treatment.

In a further aspect, the invention relates to a kit for use in the *in vitro* method for identifying a melanoma tumor suitable for treatment with the inhibitors, said kit comprising:

a) at least one reagent capable of specifically determining the expression level of the long-non coding RNA (lncRNA) LINC00518; and
b) at least one reagent capable of specifically determining the expression level of the long-non coding RNA (lncRNA) SAMMSON;
c) optionally, instructions for performing said method.

## LEGENDS TO THE FIGURES

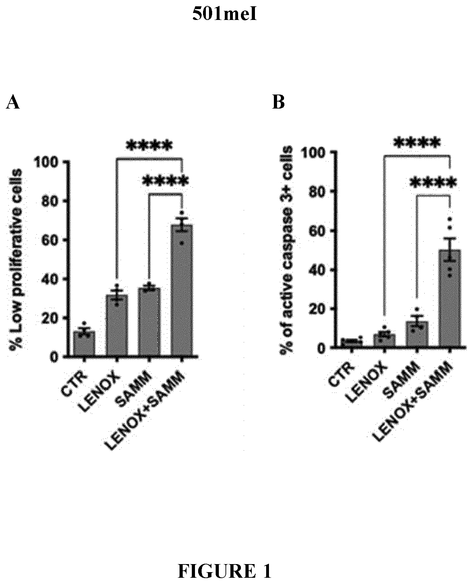

**Figure 1. LINC00518-SAMMSON co-targeting has a synergistic inhibitory effect on the cellular proliferation and survival of the 501mel melanoma cell line (melanocytic; BRAFV600E mutation).** 501mel melanoma cells transfected with suboptimal doses of LENOX (i.e. LINC00518) or SAMMSON gapmers as single agents or in pairwise combinations were incubated with the CellTrace Violet dye, cultured for 72 hours, stained with an anti-cleaved Caspase 3 antibody and analysed by flow cytometry. Percentages of low proliferative **(A)** and Active Caspase 3-positive cells **(B)** were compared between the groups by 1-way Anova.

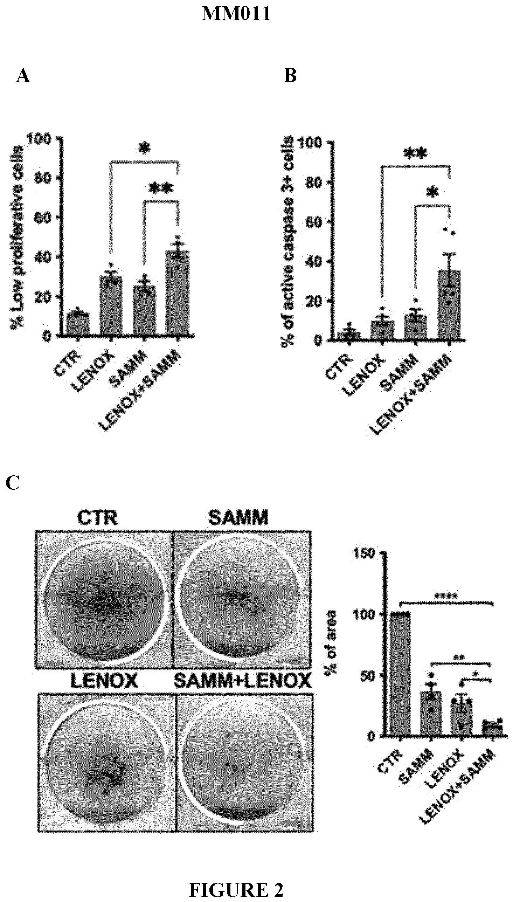

**Figure 2. LINC00518-SAMMSON co-targeting has a synergistic inhibitory effect on the cellular proliferation and survival of the MM011 melanoma cell line (melanocytic; NRAS Q61K/R mutation).** MM011 melanoma cells transfected with suboptimal doses of LENOX (i.e. LINC00518) or SAMMSON gapmers as single agents or in pairwise combinations were incubated with the CellTrace Violet dye, cultured for 72 hours, stained with an anti-cleaved Caspase 3 antibody and analysed by flow cytometry. Percentages of low proliferative (A) and Active Caspase 3-positive cells **(B)** were compared between the groups by 1-way Anova. (C) Melanoma cells were transfected as in panels (A) and (B) with CTR/LENOX/SAMMSON Gapmers, cultured for 10 days and stained with Crystal violet. Percentages of area occupied by cells in each condition were compared by 1-way Anova.

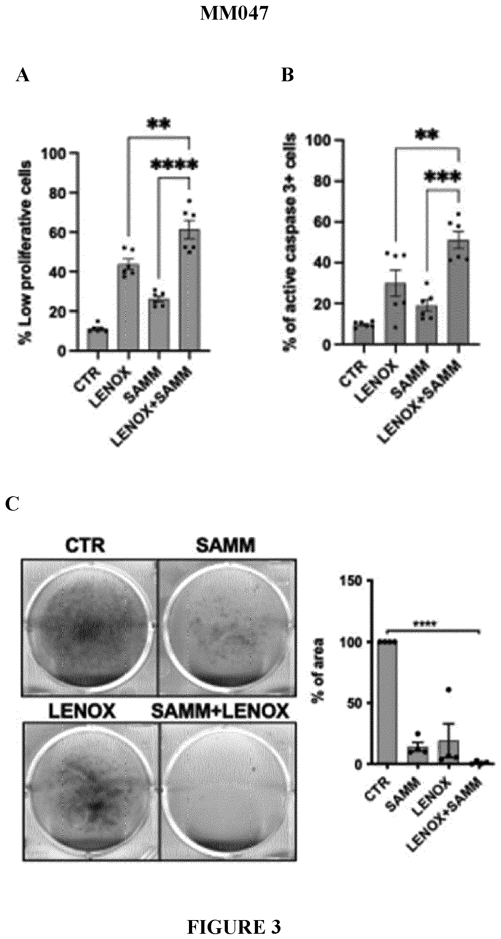

**Figure 3. LINC00518-SAMMSON co-targeting has a synergistic inhibitory effect on the cellular proliferation and survival of the MM047 melanoma cell line (mesenchymal; NRASQ61R mutation).** MM047 melanoma cells transfected with suboptimal doses of LENOX (i.e. LINC00518) or SAMMSON gapmers as single agents or in pairwise combinations were incubated with the CellTrace Violet dye, cultured for 72 hours, stained with an anti-cleaved Caspase 3 antibody and analysed by flow cytometry. Percentages of low proliferative **(A)** and Active Caspase 3-positive cells **(B)** were compared between the groups by 1-way Anova. **(C)** Melanoma cells were transfected as in panels (A) and (B) with CTR/LENOX/SAMMSON gapmers, cultured for 10 days and stained with Crystal violet. Percentages of area occupied by cells in each condition were compared by 1-way Anova.

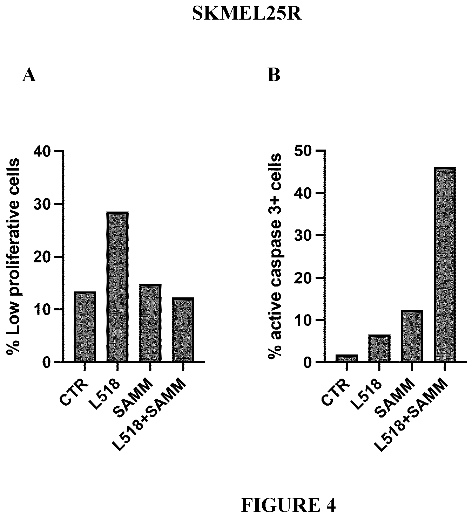

**Figure 4. LINC00518-SAMMSON co-targeting has a synergistic inhibitory effect on the cellular proliferation and survival of the SKMEL25R melanoma cell line (undifferentiated, BRAFV600E mutation; resistant to BRAF inhibitors).** Melanoma cells transfected with suboptimal doses of LENOX (i.e. LINC00518) or SAMMSON gapmers as single agents or in pairwise combinations were incubated with the CellTrace Violet dye, cultured for 72 hours, stained with an anti-cleaved Caspase 3 antibody and analysed by flow cytometry. Percentages of low proliferative **(A)** and Active Caspase 3-positive cells **(B)** were compared between the groups by 1-way Anova.

## DETAILED DESCRIPTION OF THE INVENTION

Unless otherwise defined herein, scientific and technical terms used in connection with the present invention shall have the meanings that are commonly understood by those of ordinary skill in the art. Further, unless otherwise required by context, nomenclatures used herein, and techniques are those well-known and commonly used in the art.

The present invention may be understood more readily by reference to the following detailed description, included preferred embodiments of the invention, and examples included herein.

The present invention aims at providing an innovative therapeutic approach for the treatment of melanoma, regardless of its phenotype, driver mutations or resistance to therapy, by inhibiting the functional expression of two melanoma-specific lncRNAs: LINC00518 and SAMMSON.

In a first aspect, the present invention relates to (i) an inhibitor of functional expression of the long-non coding RNA (lncRNA) LINC00518 for use in combination with (ii) an inhibitor of functional expression of the long-non coding RNA (lncRNA) SAMMSON, in the treatment of a melanoma.

In particular, the present invention is directed to the combined use of (i) an inhibitor of functional expression of the long-non coding RNA (lncRNA) LINC00518, and (ii) an inhibitor of functional expression of the long-non coding RNA (lncRNA) SAMMSON, for the preparation of a medicament for the treatment of a melanoma.

Further provided is a method for treating a melanoma, in a subject in need thereof, said method comprising the administration, to said subject, of a therapeutically effective amount of: (i) an inhibitor of functional expression of the long-non coding RNA (lncRNA) LINC00518, and (ii) an inhibitor of functional expression of the long-non coding RNA (lncRNA) SAMMSON.

A *"long-non coding RNA"* (long ncRNA, or lncRNA) is generally referred as a non-coding RNA that is at least 200 nucleotides in length. A particular class of lncRNA are *"long intergenic noncoding RNAs"* (lincRNAs), which are sequences of lncRNA transcribed from non-coding DNA sequences between protein-coding genes.

The term *"LINC00518",* also known as *"LENOX"* or" *C6orf218",* refers to the long intergenic non-protein coding RNA 518. It is disclosed in the GeneCards database under ID GC06M010428, in the HGNC database under ID 28626, in the Gene database under ID 221718, in the Ensembl database under ID ENSG00000183674 and in the Genbank database under NR_027793. The LINC00518 gene encodes 8 splicing variants (i.e. isoforms), arising from up to 4 exons (see Table 1). Unless specified otherwise, the term *"LINC00518"* encompasses all the isoforms thereof.

### Table 1. LINC00518 isoforms and exons (sequences available on Ensembl database)

| LINC00518 isoforms and exons (bp) | Ensembl reference | LINC00518 isoforms and exons (bp) | Ensembl reference |
|---|---|---|---|
| LINC00518-206 (1433 bp) Exon 1 (374 bp) | ENST00000655125.1 ENSE00003860262 | LINC00518-207 (1287 bp) Exon 1 (230 bp) | ENST00000656574.1 ENSE00003863046 |

(continued)

| LINC00518 isoforms and exons (bp) | Ensembl reference | LINC00518 isoforms and exons (bp) | Ensembl reference |
|---|---|---|---|
| Exon 2 (1059 bp) | ENSE00001848047 | Exon 2 (1057 bp) | ENSE00003886855 |
| LINC00518-205 (1729 bp) Exon 1 (491 bp) Exon 2 (179 bp) Exon 3 (1059 bp) | ENST00000496285.6 ENSE00001528640 ENSE00001528639 ENSE0000 1848047 | LINC00518-202 (1362 bp) Exon 1 (194 bp) Exon 2 (116 bp) Exon 3 (1052 bp) | ENST00000479822.2 ENSE00001852160 ENSE0000 1936862 ENSE00001882613 |
| LINC00518-208 (1709 bp) Exon 1 (316 bp) Exon 2 (116 bp) Exon 3 (226 bp) Exon 4 (1051 bp) | ENST00000662081.1 ENSE00003868168 ENSE00001936862 ENSE00001873108 ENSE00003857789 | LINC00518-204 (1228 bp) Exon 1 (305 bp) Exon 2 (179 bp) Exon 3 (226 bp) Exon 4 (518 bp) | ENST00000491317.1 ENSE00001867489 ENSE00001528639 ENSE00001873108 ENSE00001954484 |
| LINC00518-203 (999 bp) Exon 1 (233 bp) Exon 2 (766 bp) | ENST00000487130.1 ENSE00001825958 ENSE00001833540 | LINC00518-201 (853 bp) Exon 1 (248 bp) Exon 2 (605 bp) | ENST00000472178.1 ENSE00001926891 ENSE00001953166 |

The term *"SAMMSON", "LINC01212", "long intergenic non-protein coding RNA 1212",* or *"RP11-460N16.1"* as used herein refer to the long intergenic non-protein coding RNA 01212. It is disclosed in the Gene database under ID 101927152, in the HGNC database under ID 49644, in the Ensembl database under ID ENSG00000240405. The SAMMSON gene encodes 28 splicing variants (i.e. isoforms), arising from up to 10 exons (see Table 2). Unless specified otherwise, the term "*SAMMSON*" encompasses all the isoforms thereof.

Table 2. SAMMSON isoforms and exons (sequences available on Ensembl database)

| SAMMSON isoforms and exons (bp) | Ensembl reference | SAMMSON isoforms and exons (bp) | Ensembl reference |
|---|---|---|---|
| SAMMSON-227 (1199 bp) Exon 1 (22 bp) Exon 2 (242 bp) Exon 3 (153 bp) Exon 4 (90 bp) Exon 5 (90 bp) Exon 6 (77 bp) Exon 7 (65 bp) Exon 8 (103 bp) Exon 9 (71 bp) Exon 10 (286 bp) | ENST00000642114.1 ENSE00003812856 ENSE0000 1950897 ENSE00001931318 ENSE00003813611 ENSE00003813334 ENSE00003812398 ENSE00003812721 ENSE00003814215 ENSE00003814176 ENSE00003813817 | SAMMSON-214 (1264 bp) Exon 1 (38 bp) Exon 2 (242 bp) Exon 3 (153 bp) Exon 4 (90 bp) Exon 5 (104 bp) Exon 6 (192 bp) Exon 7 (74 bp) Exon 8 (70 bp) Exon 9 (301 bp) | ENST00000641395.1 ENSE00003811657 ENSE0000 1950897 ENSE00001931318 ENSE00003813611 ENSE00003812067 ENSE00003812035 ENSE00003814276 ENSE00003814012 ENSE00003814209 |
| SAMMSON-222 (2727 bp) Exon 1 (120 bp) Exon 2 (242 bp) Exon 3 (153 bp) Exon 4 (73 bp) Exon 5 (2139 bp) | ENST00000641901.1 ENSE00001953476 ENSE00001950897 ENSE00001931318 ENSE00003811698 ENSE00003812673 | SAMMSON-204 (1355 bp) Exon 1 (93 bp) Exon 2 (242 bp) Exon 3 (153 bp) Exon 4 (90 bp) Exon 5 (777 bp) | ENST00000641014.1 ENSE00003812139 ENSE0000 1950897 ENSE00001931318 ENSE00003813611 ENSE00003811799 |
| SAMMSON-203 (2168 bp) Exon 1 (80 bp) Exon 2 (162 bp) Exon 3 (242 bp) Exon 4 (153 bp) Exon 5 (90 bp) Exon 6 (1441 bp) | ENST00000641005.1 ENSE00003813525 ENSE00003813492 ENSE00001950897 ENSE00001931318 ENSE00003813611 ENSE00003811646 | SAMMSON-226 (1582 bp) Exon 1.(45 bp) Exon 2 (242 bp) Exon 3 (153 bp) Exon 4 (73 bp) Exon 5 (90 bp) Exon 6 (979 bp) | ENST00000642089.1 ENSE00003813273 ENSE0000 1950897 ENSE00001931318 ENSE00003811698 ENSE00003813611 ENSE00003813074 |

(continued)

| SAMMSON isoforms and exons (bp) | Ensembl reference | SAMMSON isoforms and exons (bp) | Ensembl reference |
|---|---|---|---|
| SAMMSON-201 (2055 bp) | ENST00000483525.2 | SAMMSON-218 (1847 bp) | ENST00000641601.1 |
| Exon 1 (135 bp) | ENSE00003812812 | Exon 1 (261 bp) | ENSE00003813550 |
| Exon 2 (242 bp) | ENSE00001950897 | Exon 2 (242 bp) | ENSE00001950897 |
| Exon 3 (153 bp) | ENSE00001931318 | Exon 3 (153 bp) | ENSE00001931318 |
| Exon 4 (1525 bp) | ENSE00001869704 | Exon 4 (1191 bp) | ENSE00003813947 |
| SAMMSON-217 (1657 bp) | ENST00000641532.1 | SAMMSON-206 (1516 bp) | ENST00000641053.1 |
| Exon 1 (71 bp) | ENSE00003811902 | Exon 1 (162 bp) | ENSE00003814113 |
| Exon 2 (242 bp) | ENSE0000 1950897 | Exon 2 (71 bp) | ENSE00003814176 |
| Exon 3 (163 bp) | ENSE00001931318 | Exon 3 (103 bp) | ENSE00003813901 |
| Exon 4 (1191 bp) | ENSE00003813947 | Exon 4 (1180 bp) | ENSE00003814292 |
| SAMMSON-211 (1645 bp) | ENST00000641286.1 | SAMMSON-213 (1521 bp) | ENST00000641336.1 |
| Exon 1 (135 bp) | ENSE00003812812 | Exon 1 (34 bp) | ENSE00003814224 |
| Exon 2 (242 bp) | ENSE00001950897 | Exon 2 (242 bp) | ENSE00001950897 |
| Exon 3 (1268 bp) | ENSE00003813536 | Exon 3 (1245 bp) | ENSE00003812390 |
| SAMMSON-221 (1546 bp) | ENST00000641715.1 | SAMMSON-212 (1487 bp) | ENST00000641295.1 |
| Exon 1 (135 bp) | ENSE00003812812 | Exon 1 (49 bp) | ENSE00003812757 |
| Exon 2 (242 bp) | ENSE00001950897 | Exon 2 (242 bp) | ENSE00001950897 |
| Exon 3 (153 bp) | ENSE00001931318 | Exon 3 (153 bp) | ENSE00001931318 |
| Exon 4 (90 bp) | ENSE00003813611 | Exon 4 (90 bp) | ENSE00003813611 |
| Exon 5 (72 bp) | ENSE00003812980 | Exon 5 (104 bp) | ENSE00003812067 |
| Exon 6 (854 bp) | ENSE00003812032 | Exon 6 (849 bp) | ENSE00003812219 |
| SAMMSON-202 (1443 bp) | ENST00000488861.7 | SAMMSON-228 (1368 bp) | ENST00000671630.1 |
| Exon 1 (55 bp) | ENSE00003881133 | Exon 1 (245 bp) | ENSE00003861233 |
| Exon 2 (242 bp) | ENSE00001950897 | Exon 2 (153 bp) | ENSE00001931318 |
| Exon 3 (153 bp) | ENSE00001931318 | Exon 3 (970 bp) | ENSE00003871684 |
| Exon 4 (993 bp) | ENSE00001958960 | | |
| SAMMSON-205 (1377 bp) | ENST00000641043.1 | SAMMSON-225 (1199 bp) | ENST00000642004.1 |
| Exon 1 (38 bp) | ENSE00003811657 | Exon 1 (44 bp) | ENSE00001861180 |
| Exon 2 (242 bp) | ENSE00001950897 | Exon 2 (242 bp) | ENSE00001950897 |
| Exon 3 (153 bp) | ENSE00001931318 | Exon 3 (153 bp) | ENSE00001931318 |
| Exon 4 (90 bp) | ENSE00003813611 | Exon 4 (90 bp) | ENSE00003813611 |
| Exon 5 (854 bp) | ENSE00003812032 | Exon 5 (670 bp) | ENSE00003813520 |
| SAMMSON-210 (1308 bp) | ENST00000641260.1 | SAMMSON-209 (1100 bp) | ENST00000641222.1 |
| Exon 1 (22 bp) | ENSE00003813264 | Exon 1 (237 bp) | ENSE00003813106 |
| Exon 2 (242 bp) | ENSE00001950897 | Exon 2 (65 bp) | ENSE00003812721 |
| Exon 3 (153 bp) | ENSE00001931318 | Exon 3 (215 bp) | ENSE00003811971 |
| Exon 4 (90 bp) | ENSE00003813611 | Exon 4 (98 bp) | ENSE00003812453 |
| Exon 5 (98 bp) | ENSE00003812541 | Exon 5 (71 bp) | ENSE00003814176 |
| Exon 6 (38 bp) | ENSE00003814220 | Exon 6 (414 bp) | ENSE00003812366 |
| Exon 7 (665 bp) | ENSE00003811871 | | |
| SAMMSON-223 (1213 bp) | ENST00000641923.1 | SAMMSON-220 (1207 bp) | ENST00000641648.1 |
| Exon 1 (27 bp) | ENSE00003813617 | Exon 1 (22 bp) | ENSE00003813264 |
| Exon 2 (242 bp) | ENSE00001950897 | Exon 2 (242 bp) | ENSE00001950897 |
| Exon 3 (153 bp) | ENSE00001931318 | Exon 3 (153 bp) | ENSE00001931318 |
| Exon 4 (73 bp) | ENSE00003811698 | Exon 4 (90 bp) | ENSE00003813611 |

(continued)

| SAMMSON isoforms and exons (bp) | Ensembl reference | SAMMSON isoforms and exons (bp) | Ensembl reference |
|---|---|---|---|
| Exon 5 (90 bp) | ENSE00003813611 | Exon 5 (72 bp) | ENSE00003812980 |
| Exon 6 (628 bp) | ENSE00003812882 | Exon 6 (628 bp) | ENSE00003812882 |
| SAMMSON-207 (1169 bp) | ENST00000641128.1 | SAMMSON-219 (1162 bp) | ENST00000641635.1 |
| Exon 1 (134 bp) | ENSE00003811838 | Exon 1 (49 bp) | ENSE00003812757 |
| Exon 2 (242 bp) | ENSE00001950897 | Exon 2 (242 bp) | ENSE00001950897 |
| Exon 3 (153 bp) | ENSE00001931318 | Exon 3 (153 bp) | ENSE00001931318 |
| Exon 4 (90 bp) | ENSE00003813611 | Exon 4 (90 bp) | ENSE00003813611 |
| Exon 5 (550 bp) | ENSE00003812405 | Exon 5 (628 bp) | ENSE00003812882 |
| SAMMSON-216 (1110 bp) | ENST00000641457.1 | SAMMSON-215 (1046 bp) | ENST00000641431.1 |
| Exon 1 (135 bp) | ENSE00003812812 | Exon 1 (65 bp) | ENSE00003812570 |
| Exon 2 (242 bp) | ENSE00001950897 | Exon 2 (242 bp) | ENSE00001950897 |
| Exon 3 (153 bp) | ENSE00001931318 | Exon 3 (153 bp) | ENSE00001931318 |
| Exon 4 (90 bp) | ENSE00003813611 | Exon 4 (73 bp) | ENSE00003811698 |
| Exon 5 (490 bp) | ENSE00003811691 | Exon 5 (513 bp) | ENSE00003812178 |
| SAMMSON-208 (868 bp) | ENST00000641194.1 | SAMMSON-224 (853 bp) | ENST00000641929.2 |
| Exon 1 (84 bp) | ENSE00003812068 | Exon 1 (64 bp) | ENSE00003814172 |
| Exon 2 (242 bp) | ENSE00001950897 | Exon 2 (242 bp) | ENSE00001950897 |
| Exon 3 (153 bp) | ENSE00001931318 | Exon 3 (153 bp) | ENSE00001931318 |
| Exon 4 (389 bp) | ENSE00003811935 | Exon 4 (394 bp) | ENSE00003811601 |

By "*functional expression*" of a gene, it is meant the transcription and/or translation of functional gene product.

An *"inhibitor of functional expression"* of a gene refers to a molecule or technical means capable of decreasing or even abolish the expression of said gene. For non-protein coding genes like LINC00518 and SAMMSON, functional expression can be deregulated on at least two levels: first, at the DNA level, e.g. by absence or disruption of the gene, or lack of transcription taking place (in both instances preventing synthesis of the relevant gene product); second, at the RNA level, e.g. by lack of splicing or lack or decrease in the activity mediated by said lncRNA. Inhibition can be evaluated by any means known to those skilled in the art including, but not limited to, assessing the level of lncRNA transcript using e.g. quantitative PCR. Besides, in the context of the present invention, it shall be understood that the inhibitor is selective, or in other words, specific for the targeted lncRNA, in that it does not inhibit, or at least does not substantially inhibit, any other target.

An inhibitor according to the invention is capable of inhibiting the functional expression of the lncRNA *in vivo* and/or *in vitro.* The inhibitor may inhibit the functional expression of the lncRNA by at least about 10%, 15% 20%, or 25%, preferably by at least about 30%, 35%, 40% or 45%, still preferably by at least about 50%, 55%, 60%, or 65%, yet preferably by at least about 70%, 75%, 80%, or 85%, more preferably by at least about 90%, or 95%.

Generally speaking, the term *"treatment"* or *"treating"* means obtaining a desired physiological or pharmacological effect depending on the degree of severity of the symptom or disorder of interest, or risks thereof, i.e. herein, depending on the degree of severity or risks of developing such symptom or disorder. In the context of cancer such as melanoma, this includes, *inter alia,* the alleviation of symptoms, the reduction of inflammation, the inhibition of cancer cell growth, and/or the reduction of tumor size. Furthermore, these terms are intended to encompass curing as well as ameliorating at least one symptom of the condition or disease. For example, in the case of cancer, a response to treatment includes a reduction in cachexia, increase in survival time, elongation in time to tumor progression, reduction in tumor mass, reduction in tumor burden and/or a prolongation in time to tumor metastasis, time to tumor recurrence, tumor response, complete response, partial response, stable disease, progressive disease, progression free survival, overall survival, each as measured by standards set by the National Cancer Institute and the U.S. Food and Drug Administration for the approval of new drugs. See Johnson et al., J. Clin. Oncol., 2009; 21(7): 1404-1411.

In a preferred embodiment, the term treatment refers to the inhibition or reduction of cancer cell proliferation and/or the increase in cancer cell apoptosis in a subject having the cancer, herein melanoma.

In a more preferred embodiment, the inhibition or reduction of cancer cells and/or the increase in apoptosis of cancer cells is independent of the status of tumor-associated proteins, herein independent of BRAF and/or NRAS status.

The term *"status"* as used herein with regard to a particular protein, specifically tumor-associated proteins (e.g.

BRAF status, NRAS status), refers to the mutational status and/or the expression of these particular proteins in cells of interest (herein, melanoma cells). Typically, the term is used in the sense "*irrespective of*" or "*independent of*" status, meaning that an effect is observed irrespective of expression levels of, or presence of mutations in, the particular protein in the cells of interest (herein, melanoma cells).

In melanoma, BRAF mutations are more commonly observed in intermittently sun-exposed skin, and associated with KIT mutations, which are present predominantly in mucosal and acral melanomas. Examples of BRAF mutations associated with melanoma include, without limitation, R461I, I462S, G463E, G463V, G465A, G465E, G465V, G468A, G468E, N580S, E585K, D593V, F594L, G595R, L596V, T598I, V599D, V599E, V599K, V599R, V600E/K (the most prominent), K600E, A727V, and most of these mutations are clustered to two regions: the glycine-rich P loop of the N lobe and the activation segment and flanking regions. In a particular embodiment, the BRAF mutation is V600E/K, preferably V600E.

In melanoma, NRAS mutations are more commonly observed in older patients (above 55 years-old), with a chronic pattern of UV exposure with lesions are usually located at the extremities. Examples of NRAS mutations typically associated with melanoma include, without limitation, G12D/C, G13R, Q61K, Q61R, Q61L, Q61V, Q61H, Q61P. In a particular embodiment, the NRAS mutation is Q61K/R.

It shall nevertheless be understood that BRAF and NRAS co-mutations are not mutually exclusive.

A "*therapeutically effective amount*" means herein an amount that is sufficient to achieve the effect for which it is indicated, herein the treatment of a melanoma. The amount of the combination therapy of the invention to be administered can be determined by standard procedures well known by those of ordinary skill in the art. Physiological data of the patient (e.g. age, size, and weight), the routes of administration and the disease to be treated have to be taken into account to determine the appropriate dosage, optionally compared with subjects that do not suffer from a melanoma. The amount may also vary according to other components of a treatment protocol (e.g. administration of other medicaments, etc.).

A "*subtherapeutic amount*" or "*subtherapeutic dose*" is an amount that is not therapeutically effective if administered in absence of any other agent or therapy.

It should be further understood that the "*subject*" or "*patients*" to be treated according to the invention is one having an integumentary system. Accordingly, preferred subjects to be treated are animals, more preferably mammals, most preferably humans.

The subject to be treated has a melanoma. A "*melanoma*", also known as "*malignant melanoma*", refers to a type of cancer that develops from the pigment-producing cells called melanocytes. There are three main categories of melanoma: cutaneous melanoma, the most common, which corresponds to the melanoma of the skin; mucosal melanoma which can affect any mucous membrane of the body, including the nasal passages, throat, vagina, anus, or mouth; and ocular melanoma, which is a rare form of melanoma affecting the eye (also known as uveal or choroidal melanoma).

The melanoma to be treated can be at any stage. For instance, the melanoma can be at stage I/II, II, III, or IV, preferably stage II, III, or IV, more preferably stage III, or IV. More specifically, the melanoma can be at stage T1a, T1b, T2a, T2b, T3a, T4a, T4b, N1, N2, N3, M1a, M1b, or M1c, preferably stage T2b, T3a, T4a, T4b, N1, N2, N3, M1a, M1b, or M1c. In a preferred embodiment, the melanoma is at an advanced or metastatic stage.

The melanoma can be preferably selected from the group consisting of lentigo maligna, lentigo maligna melanoma, superficial spreading melanoma, acral lentiginous melanoma, mucosal melanoma, nodular melanoma, polypoid melanoma, desmoplastic melanoma, melanoma with small nevus-like cells, melanoma with features of a Spitz nevus, uveal melanoma, Harding-Passey melanoma, juvenile melanoma, amelanotic melanoma, Cloudman's melanoma, and vaginal melanoma.

The subject to be treated may have resistant melanoma. As used herein, the term "*resistant melanoma*" refers to a melanoma, which does not respond to a conventional treatment for said cancer. The cancer may be resistant at the beginning of treatment or it may become resistant during treatment or does not respond anymore. The resistance to the treatment may lead to rapid progression of metastatic of melanoma.

The melanoma can be resistant to a targeted therapy, especially inhibitors of the MAPK pathway which include BRAF inhibitors, MEK inhibitors and C-Kit inhibitors; to chemotherapy; and/or to immune checkpoint inhibitors.

In a preferred embodiment, the melanoma is resistant to a targeted therapy, especially BRAF inhibitors such as dabrafenib, vemurafenib, or encorafenib, and/or MEK inhibitors, such as trametinib. The inhibitors of functional expression according to the invention may indeed restore or increase the sensitivity of melanoma to this type of therapy.

Alternatively, the melanoma may not be resistant to a targeted therapy, especially inhibitors of the MAPK pathway which include BRAF inhibitors, MEK inhibitors and C-Kit inhibitors; to chemotherapy; and/or to immune checkpoint inhibitors.

In a preferred embodiment, the melanoma is not resistant to a targeted therapy, especially BRAF inhibitors such as dabrafenib, vemurafenib, or encorafenib, and/or MEK inhibitors, such as trametinib. The inhibitors of functional expression according to the invention may indeed prevent, decrease or delay the appearance of a resistance to such therapy.

In a preferred embodiment, whether the melanoma is resistant or not, the melanoma can be associated with a BRAF mutation and/or with a NRAS mutation, as described above. The inhibitors of functional expression according to the invention may indeed act independently of BRAF and/or NRAS status, as explained above.

The present invention requires a combined inhibition of two melanoma-specific lncRNAs: LINC00518 and SAMMSON.

Inhibitors of functional expression of LINC00518 and SAMMSON have been reported in the scientific and patent literature (WO2015024986; Leucci et al., Nature, 2016: 531:518-22; WO2021/152005; the contents of which are incorporated herein by reference in their entireties). Below is provided a non-exhaustive list of such inhibitors which can be used in the present invention.

Given the nature of the targeted RNA, each of the inhibitors of functional expression used in the present invention can preferably be either a nucleic acid molecule interfering specifically with the expression of the long-non coding RNA, or a genome editing system comprising a nuclease engineered to target specifically the long-non coding RNA.

A nucleic acid molecule interfering specifically with the expression of the long-non coding RNA might be preferred should one wish to inhibit functional expression at the RNA level, while a genome editing system comprising a nuclease engineered to target specifically the long-non coding RNA might be preferred should one wish to inhibit functional expression at the DNA level.

In a preferred embodiment, the inhibitor used in the present invention can be a nucleic acid molecule interfering specifically with the expression of the long-non coding RNA.

As used throughout the specification, the terms *"nucleic acid molecule"*, *"nucleic acid"*, *"polynucleotide"* (polynucleoside) or *"oligonucleotide"* (oligonucleoside) refers to a succession of natural or synthetic nucleotides (or nucleosides) connected by internucleotide (or internucleoside) linkages, wherein each nucleotide (or nucleoside) or internucleotide (or internucleoside) linkages may be modified or unmodified. A nucleotide is comprised of a nucleoside and a phosphate group. A nucleoside is comprised of a nucleobase and a sugar. A nucleobase comprises a modified and unmodified nucleobase. Unmodified nucleobases are well-known in the art and include adenine (A), thymine (T), cytosine (C), uracil (U) and guanine (G). An internucleotide (internucleoside) linkage is a bond that forms a covalent linkage between adjacent nucleotides (nucleosides) in a nucleic acid, such as phosphate internucleotide linkages that are naturally occurring. A nucleic acid may be a single-stranded or double-stranded DNA such as cDNA, genomic DNA, ribosomal DNA, and the transcription product of said DNA, such as RNA. Nucleic acids also encompass nucleic acids which can hybridize to a nucleic acid of reference. When a nucleic acid is designed to hybridize to a nucleic acid of reference, it can be chemically modified to enhance its stability, nuclease resistance, target specificity and/or improve their pharmacological properties (e.g. reduced toxicity, increased intracellular transport, etc.). For example, a nucleic acid may comprise modified nucleotide(s) and/or backbone, such as a modified sugar, a modified nucleobase, and/or a modified internucleotide (internucleoside) linkage. A standard modification is the replacement of native phosphates of the internucleotide (internucleoside) linkage with phosphorothioates (PS) so as to reduce the sensitivity to nucleases and accordingly improve the stability, half-life and tissue distribution of the nucleic acid (Eckstein F., Antisense and Nucleic Acid Drug Development, 2000, 10(2): 1117-221). Another standard modification which increases the affinity of the nucleic acid towards its target is the incorporation in the sugar moiety of a methoxyethyl (MOE) or a constrained ethyl (cEt) to the 2'position, or the tethering of the 4'-carbon to the 2-hydroxyl of the ribose ring to create a bicyclic locked nucleic acid (LNA), to name a few. All these modications are well known to the one skilled in the art (see Watts et al. J Pathol., 2012; 226(2):365-379; Seth et al., J Clin Invest., 2019; 129(3): 915-925).

Preferred nucleic acid molecules interfering specifically with the expression of the long-non coding RNA according to the invention are those capable of hybridizing specifically to the gene or transcripts of the lncRNA, at least to a part thereof; as such, these are usually non-naturally occurring nucleic acids (i.e. synthetic).

*"Hybridizing"* or *"hydridization"* means the pairing or annealing to a target, herein under physiological conditions, typically via hydrogen bonding between complementary nucleotides, such as Watson-Crick, Hoogsteen or reversed Hoogsteen hydrogen bonding. A specific hybridization therefore means that the pairing or annealing is specific to the target (no off-targets effects, or at least no substantial off-targets effects).

*"A nucleic acid molecule capable of hybridizing"* to a target nucleic acid thus means that a stretch of this nucleic acid is capable of forming base pairs to another stretch of the target nucleic acid. It is thus not absolutely required that all the bases in the region of complementarity are capable of pairing with bases in the opposing strand. Mismatches may be tolerated to some extent, as long as in the circumstances, the stretch of nucleotides is capable of hybridizing to its complementary part.

The nucleic acid molecule interfering specifically with the expression of the long-non coding RNA is preferably an antisense nucleic acid, an RNAi nucleic acid, or a ribozyme.

The term *"antisense nucleic acid"* or *"antisense oligonucleotides"* (ASO) designates a synthetic single-stranded oligonucleotide of which the sequence is at least partially complementary to a target nucleic acid, such as to the RNA sequence of a target gene (Lee et al., J Cardiovasc Transl Res. 2013; 6(6):969-80; DeVos et al., Neurotherapeutics 2013; 10(3):486-972013). An antisense nucleic acid is capable of altering the expression of a specific target gene, either by splicing modification, or by recruiting RNAse H leading to RNA degradation of RNA-DNA duplexes, thus blocking the expression of the target gene. An antisense nucleic acid is typically short in length, in general 5 to 50 nucleotides in length, such as 5, 10, 15, 20, 25, 30, 35, 40, 45 or 50 nucleotides in length, more preferably 10, 15, 20, 25, 30, 35 nucleotides in length. It is well within the skill of the person in the art to design antisense nucleic acids specific to a target, based on the knowledge of a target sequence such as introns, exons, or 5'CAP (DeVos et al., Neurotherapeutics 2013; 10(3):486-972013). Antisense nucleic acids can be prepared by methods well-known in the art, such as by chemical synthesis and enzymatic ligation reactions.

In a preferred embodiment, the antisense nucleic acid interfering specifically with the expression of the long-non coding RNA inhibits the splicing of the pre- long-non coding RNA, or in other words, it inhibits the formation of the mature long-non coding RNA. An antisense nucleic acid can indeed be designed to block a splice acceptor (SA) site and/or an exon splicing enhancer (ESE) and/or any sequence which could modulate a pre-RNA splicing, i.e. it can be designed to be complementary to a part of the pre-RNA comprising an SA, an ESE, or any sequence which could modulate its splicing.

In an alternative preferred embodiment, the antisense nucleic acid interfering specifically with the expression of the long-non coding RNA induces a RNAse H mediated degradation. RNAse H is a cellular enzyme which recognizes duplex between DNA and RNA, and enzymatically cleaves the RNA molecules. Accordingly, to induce RNAse H mediated degradation, the antisense nucleic acid interfering specifically with the expression of the long-non coding RNA comprises a region that comprises DNA or DNA-like nucleotides complementary to the targeted lncRNA which is responsible for RNAse H recruitment, ultimately leading to the cleavage of the target nucleic acid.

Regardless of its mode of action for inhibiting the functional expression of the lncRNA, the antisense nucleic acid interfering specifically with the expression of the long-non coding RNA is complementary to all or part of the lncRNA, preferably to a part thereof, such as any one of its exons or introns. Sequences of LINC00518 and SAMMSON are well known in the art, as explained above.

In a preferred embodiment, the antisense nucleic acid interfering specifically with the expression of LINC00518 comprises or consists of a contiguous nucleotide sequence of at least 10 to 30 (preferably 11 to 29, 12 to 26, 13 to 24, 14 to 22, 14 to 17, 14, 15 or 16) nucleotides in length that is complementary to a specific region of the LINC00518 sequence. Non limiting examples of such antisense nucleic acids include, without limitation, those comprising or consisting a contiguous nucleotide sequence of at least 10 nucleotides in length of the sequence SEQ ID NO: 2 or 3, especially SEQ ID NO: 2, such as those comprising or consisting of the sequence SEQ ID NO: 2 or 3, especially SEQ ID NO: 2.

In a preferred embodiment, the antisense nucleic acid interfering specifically with the expression of SAMMSON comprises or consists of a contiguous nucleotide sequence of at least 10 to 30 (preferably 11 to 29, 12 to 26, 13 to 24, 14 to 22, 14 to 17, 14, 15 or 16) nucleotides in length that is complementary to a specific region of the SAMMSON sequence. Non limiting examples of such antisense nucleic acids include, without limitation, those comprising or consisting a contiguous nucleotide sequence of at least 10 nucleotides in length of the sequence SEQ ID NO: 4, preferably those comprising or consisting of the sequence SEQ ID NO: 4.

As described above, the antisense nucleic acid interfering specifically with the expression of the long-non coding RNA may further comprise at least one modified sugar, at least one modified internucleotide (or internucleoside) linkage, and/or at least one modified nucleobase, so as to enhance its properties, such a reduction in sensitivity to nucleases, increase in stability, half-life and/or tissue distribution, and/or enhancement of its affinity towards its target nucleic acid.

In a preferred embodiment, the antisense nucleic acid interfering specifically with the expression of the long-non coding RNA comprises at least one modified sugar, in particular in position 2' of the nucleotide (or nucleoside) such as 2'-O-methyl (2'-O-Me), 2'fluoro (2'-F), 2'O-methoxyethyl (2'-MOE also known as MOE), 2',4'-brigded, or cEt (constrained ethyl).

A "modified sugar" is a sugar that is non-naturally occurring. 2',4'-brigded, which is the introduction of a methylene bridge between the 2' and 4' position of the nucleotide, defining locked nucleic acids (LNA), is herein particularly preferred so as to confer or increase the stability of a nucleic acid when hybridized to a complementary DNA or RNA nucleic acid.

In a preferred embodiment, the antisense nucleic acid interfering specifically with the expression of the long-non coding RNA comprises at least one modified internucleotide (or internucleoside) linkage, such as a phosphorothioate internucleotide (or internucleoside) linkage.

A "modified internucleotide linkage" (or modified internucleoside linkage) is an internucleotide (or internucleoside) linkage in a nucleic acid that is non-naturally occurring, and is thus referred as a non-phosphate linkage. A phosphorothioate linkage, which is a modified phosphate linkage in which one of the non-bridging oxygen atoms is replaced with a sulfur atom, is herein particularly preferred so as to confer or increase the resistance of a nucleic acid to nucleases.

In a preferred embodiment, the antisense nucleic acid interfering specifically with the expression of the long-non coding RNA is a gapmer.

A "gapmer" refers to a nucleic acid molecule comprising an internal segment having a plurality of nucleotides (or nucleosides) that support RNase H cleavage positioned between external segments, each having one or more nucleotides (or nucleosides), wherein the nucleotide (or nucleosides) comprising the internal segment are chemically distinct from the immediately adjacent nucleotide(s) (or nucleoside(s)) comprising the external segments. The internal or central segment may be referred to as the *"gap"*, *"gap segment"* or *"gap region"* (G); while the external segments may be referred to as the *"wings"*, *"flanks"*, *"wing segments"*, *"flank segments"*, *"wing regions"* or *"flank regions"* (F for the 5' flank region and F' for the 3' flank region). Typically, the F and F' regions are composed of modified ribonucleotides (RNA*) which are complementary to a target nucleic acid; whereas the G region is composed of deoxyribonucleotides, i.e. DNA or DNA-like molecules, which is responsible for RNAse H recruitment which ultimately leads to the degradation of the target nucleic acid. A gapmer is therefore a chimeric antisense. Gapmers can typically comprise a gap region (G) of 5 to 15 deoxynucleotides (or deoxynucleosides) flanked by wing regions (F and F') of 2 to 10 modified nucleotides (or nucleosides) each.

In a preferred embodiment, the gapmer interfering specifically with the expression of the long-non coding RNA comprises or consists of a contiguous nucleotide (or nucleoside) sequence corresponding to the following formula:

5'-F (RNA*) - G (DNA or DNA-like) – F' (RNA*)-3'

It should be understood that any of the modifications that can improve the properties of the gapmer, as discussed above, can be used and combined together.

For example, the gapmer interfering specifically with the expression of the long-non coding RNA can comprise or consist of a contiguous nucleotide (or nucleoside) sequence that is complementary to a specific region of the lncNRA sequence and that corresponds to the following formula:

5'-F (RNA*) - G (DNA or DNA-like) – F' (RNA*)-3'

wherein the gap segment (G) comprises or consists of a sequence of at least 5 to 15 nucleotides (or nucleosides) in length,

wherein each wing segment (F or F') comprises at least 2 to 10 nucleotides (or nucleosides) in length,
wherein each nucleotide (or nucleoside) of each wing segment (F and F') comprises a modified sugar, in particular in position 2' of the nucleotide (or nucleoside) such as 2'-O-methyl (2'-O-Me), 2'fluoro (2'-F), 2'O-methoxyethyl (2'-MOE also known as MOE), 2',4'-brigded, or cEt (constrained ethyl), more preferably 2',4'-brigded, and
wherein each internucleotide (or internucleoside) linkage is a modified linkage, preferably is a phosphorothioate linkage.

In a preferred embodiment, the gapmer interfering specifically with the expression of LINC00518 comprises or consists of a contiguous nucleotide (or nucleoside) sequence that is complementary to a specific region of the LINC00518 sequence and that corresponds to the following formula:

5'-F (RNA*) - G (DNA or DNA-like) – F' (RNA*)-3'

wherein the gap segment (G) comprises or consists of a sequence of at least 5 to 15 nucleotides (or nucleosides) in length,

wherein each wing segment (F or F') comprises at least 2 to 10 nucleotides (or nucleosides) in length,
wherein each nucleotide (or nucleoside) of each wing segment (F and F') comprises a modified sugar, in particular in position 2' of the nucleotide (or nucleoside) such as 2'-O-methyl (2'-O-Me), 2'fluoro (2'-F), 2'O-methoxyethyl (2'-MOE also known as MOE), 2',4'-brigded, or cEt (constrained ethyl), more preferably 2',4'-brigded, and
wherein each internucleotide (or internucleoside) linkage is a modified linkage, preferably is a phosphorothioate linkage.

Non limiting examples of gapmers interfering specifically with the expression of LINC00518 include, without limitation, gapmers comprising or consisting of any one of the following sequences: Cbs Cbs Gbs dAs mdCs mdCs dTs dGs dAs dAs dTs dTs dGs Cbs Abs Ab (SEQ ID NO: 5), or Gbs Tbs Abs dGs dAs dGs dGs mdCs dTs dAs dGs dAs dAs Cbs Tbs Gb (SEQ ID NO: 6), wherein A is an adenine, mC is a 5-methylcytosine, G is a guanine, T is a thymine, b is LNA (2',4'-methylene bridged sugar moiety), d is a 2'-deoxyribosyl sugar moiety, and s is a phosphorothioate internucleotide (or internucleoside) linkage.

In a preferred embodiment, the gapmer interfering specifically with the expression of SAMMSON comprises or consists of a contiguous nucleotide (or nucleoside) sequence corresponding to the following formula:

## 5'-F (RNA\*) - G (DNA or DNA-like) – F' (RNA\*)-3'

wherein the gap segment (G) comprises or consists of a sequence of at least 5 to 15 nucleotides (or nucleosides) in length that is complementary to a specific region of the SAMMSON sequence,

wherein each wing segment (F or F') comprises at least 2 to 10 nucleotides (or nucleosides) in length,
wherein each nucleotide (or nucleoside) of each wing segment (F and F') comprises a modified sugar, in particular in position 2' of the nucleotide (or nucleoside) such as 2'-O-methyl (2'-O-Me), 2'fluoro (2'-F), 2'O-methoxyethyl (2'-MOE also known as MOE), 2',4'-brigded, or cEt (constrained ethyl), more preferably 2',4'-brigded, and
wherein each internucleotide (or internucleoside) linkage is a modified linkage, preferably is a phosphorothioate linkage.

Non limiting examples of gapmers interfering specifically with the expression of SAMMSON include, without limitation, gapmers comprising or consisting of the following sequence: Gbs Tbs Gbs dTs dGs dAs dAs dmCs dTs dTs dGs Gbs Cbs Tb (SEQ ID NO: 7), wherein A is an adenine, mC is a 5-methylcytosine, G is a guanine, T is a thymine, b is LNA (2',4'-methylene bridged sugar moiety), d is a 2'-deoxyribosyl sugar moiety, and s is a phosphorothioate internucleotide (or internucleoside) linkage.

Other formats of gapmers are well-known in the art, and can be easily conceived by the skilled practitioner. See for example WO2021/152005, incorporated herein in its entirety.

In another embodiment, RNAi can be used to inhibit the functional expression of the lncRNA.

"RNAi nucleic acid" refers to a nucleic acid that can inhibit expression of a target gene by RNA interference (RNAi) mechanism. By contrast to antisense nucleic acids, RNAi nucleic acids target mature RNA. RNAi nucleic acids are well-known in the art, and include short-hairpin RNA (shRNA), small interfering RNA (siRNA), double-stranded RNA (dsRNA), and single-stranded RNA (ssRNA) (Sohail et al. 2004, Gene Silencing by RNA Interference: Technology and Application 1st Edition, ISBN 9780849321412; WO 99/32619; Wang et al., Pharm Res 2011, 28:2983-2995). RNA interference designates a phenomenon by which dsRNA specifically suppresses expression of a target gene at post-transcriptional level. In normal conditions, RNA interference is initiated by double-stranded RNA molecules (dsRNA) of several thousands of base pair length. *In vivo,* dsRNA - such as shRNA- introduced into a cell is cleaved by Dicer into a mixture of short interfering RNA called siRNA; the latter will bind to another enzyme (RISC) that will catalyze the cleavage of both the siRNA and target mRNA (Bernstein et al. Nature. 2001;409(6818):363-6). In mammalian cells, the siRNAs that are naturally produced by Dicer are typically 21-23 bp in length, with a 19 or 20 nucleotides duplex sequence, two-nucleotide 3' overhangs and 5'-triphosphate extremities (Zamore et al. Cell. 2000,101(1):25-33; Elbashir et al. Genes Dev. 2001, 15(2): 188-200; Elbashir et al. EMBO J. 2001, 20(23):6877-88). The selected siRNA or shRNA target sequence should be subjected to a BLAST search against EST database to ensure that the only desired gene is targeted. Various products are commercially available to aid in the preparation and use of synthetic siRNA or shRNA. The RNAi nucleic acid can be of at least about 10 to 40 nucleotides (or nucleosides) in length, preferably about 15 to 30 base nucleotides (or nucleosides) in length. siRNA or shRNA can comprise naturally occurring RNA, synthetic RNA, or recombinantly produced RNA, as well as altered RNA that differs from naturally-occurring RNA by the addition, deletion, substitution and/or alteration of one or more nucleotides (or nucleosides). Such alterations can include addition of non-nucleotide (or non-nucleoside) material, such as to the end of the molecule or to one or more internal nucleotides (or nucleoside) of the RNAi, including modifications that make the RNAi resistant to nuclease digestion, as described above.

Thus, in a preferred embodiment, the nucleic acid molecule interfering specifically with functional expression of the lncRNA is an RNAi nucleic acid, that is complementary to at least one part of the lncRNA, in particular to at least one exon thereof. The RNAi nucleic acid can be a siRNA or shRNA of at least about 10 to 40 nucleotides (or nucleosides) in length, preferably of about 15 to 30 nucleotides (or nucleosides) in length.

In a preferred embodiment, the RNAi nucleic acid interfering specifically with the expression of LINC00518 comprises or consists of a contiguous nucleotide sequence of at least 10 to 40 (preferably 15 to 30, 16 to 25, 17 to 24, 20, 21, 22 or 23) nucleotides in length that is complementary to a specific region of the LINC00518 sequence. Non limiting examples of such RNAi nucleic acid include, without limitation, those comprising or consisting a contiguous nucleotide sequence of at least 10 nucleotides in length of the sequence SEQ ID NO: 9, preferably those comprising or consisting of the sequence SEQ ID NO: 9.

In a preferred embodiment, the RNAi nucleic acid interfering specifically with the expression of SAMMSON comprises or consists of a contiguous nucleotide sequence of at least 10 to 40 (preferably 15 to 30, 16 to 25, 17 to 24, 20, 21, 22 or 23) nucleotides in length that is complementary to a specific region of the SAMMSON sequence. Non limiting examples of such RNAi nucleic acid include, without limitation, those comprising or consisting a contiguous nucleotide sequence of at least 10 nucleotides in length of the sequence SEQ ID NO: 10, preferably those comprising or consisting of the sequence SEQ ID NO: 10.

In another embodiment, the nucleic acid interfering specifically with the functional expression of the lncRNA is a ribozyme.

*"Ribozymes"* are catalytic RNA molecules with ribonuclease activity which are capable of cleaving a single-stranded nucleic acid, such as an mRNA, to which they have a complementary region. Ribozyme molecules specific for a target (herein a lncRNA) can be designed, produced, and administered by methods commonly known to the art (see e.g., Fanning and Symonds (2006) RNA Towards Medicine (Handbook of Experimental Pharmacology), ed. Springer p. 289-303).

In a preferred embodiment, the nucleic acid molecule interfering specifically with the functional expression of LINC00518 is a ribozyme targeting said lncRNA.

In a preferred embodiment, the nucleic acid molecule interfering specifically with the functional expression of SAMMSON is a ribozyme targeting said lncRNA.

Yet, in another embodiment, genome editing can be used to inhibit the functional expression of the lncRNA. More specifically, one can use a genome editing system comprising a nuclease engineered to target the lncRNA gene.

*"Genome editing"* is a type of genetic engineering in which DNA is inserted, replaced, or removed from a genome using artificially engineered nucleases, also called molecular scissors. Nucleases create specific double-stranded break (DSBs) at desired locations in the genome, and harness the cell's endogenous mechanisms to repair the induced break by natural processes of homologous recombination (HR) or non-homologous end-joining (NHEJ). There are currently four known families of nucleases suitable for genome editing: zinc finger nucleases (ZFNs), transcription activator-like effector nucleases (TALENs), the CRISPR/Cas system in particular the Cas9 system (Mali et al, Nature Methods, 2013;10(10):957-63), or engineered meganucleases re-engineered homing endonucleases. Said nucleases can be delivered to the cells either as DNAs or mRNAs, such DNAs or mRNAs and can be engineered to target the lncRNA gene.

A particularly preferred genome editing system according to the invention is the CRISPR/Cas system in particular the Cas9 system.

In a preferred embodiment, the genome editing system interfering specifically with functional expression of LINC00518 is a CRISPR/Cas system, in particular a CRISPR/Cas9 system, targeting said lncRNA. To do so, one may use one or more single guide RNA (sgRNA) targeting specifically LINC00518. Non limiting examples of such sgRNA include, without limitation, those comprising or consisting a contiguous nucleotide sequence of at least 10 nucleotides in length of the sequence SEQ ID NO: 11, 12, or 13, preferably those comprising or consisting of the sequence SEQ ID NO: 11, 12, or 13, or any combination thereof. In a preferred embodiment, the genome editing system is a triplet-target CRISPR system, meaning that three guide RNA are used to target LINC00518, such as those described above.

In a preferred embodiment, the genome editing system interfering specifically with functional expression of SAMMSON is a CRISPR/Cas system, in particular a CRISPR/Cas9 system, targeting said lncRNA. To do so, one may use one or more single guide RNA (sgRNA) targeting specifically SAMMSON.

The skilled person would readily understand that any one of the (i) inhibitors of functional expression of the long-non coding RNA (lncRNA) LINC00518, and any one of the (ii) inhibitors of functional expression of the long-non coding RNA (lncRNA) SAMMSON, as described herein, can be combined for the purpose of the present invention.

In a particularly preferred embodiment, (i) the inhibitor of functional expression of the long-non coding RNA (lncRNA) LINC00518 is a nucleic acid molecule interfering specifically with the expression of said lncRNA, as described above; and (i) the inhibitor of functional expression of the long-non coding RNA (lncRNA) SAMMSON is a nucleic acid molecule interfering specifically with the expression of said lncRNA, as described above.

In a further preferred embodiment, (i) the nucleic acid molecule interfering specifically with the expression of LINC00518 is an antisense nucleic acid, as described above; and (ii) the nucleic acid molecule interfering specifically with the expression of SAMMSON is an antisense nucleic acid, as described above.

For example, (i) the nucleic acid molecule interfering specifically with the expression of LINC00518 can be an antisense nucleic acid comprising or consisting a contiguous nucleotide sequence of at least 10 nucleotides in length of the sequence SEQ ID NO: 2 or 3, especially SEQ ID NO: 2, such as an antisense nucleic acid comprising or consisting of the sequence SEQ ID NO: 2 or 3, especially SEQ ID NO: 2; and (ii) the nucleic acid molecule interfering specifically with the expression of SAMMSON can be an antisense nucleic acid comprising or consisting a contiguous nucleotide sequence of at least 10 nucleotides in length of the sequence SEQ ID NO: 4, such as an antisense nucleic acid comprising or consisting of the sequence SEQ ID NO: 4.

In a more preferred embodiment, (i) the antisense nucleic acid interfering specifically with the expression of LINC00518 is a gapmer, as described above; and (ii) the antisense nucleic acid interfering specifically with the expression of SAMMSON is a gapmer, as described above.

Yet, in a further preferred embodiment, the gapmer interfering specifically with the expression of LINC00518 is an LNA gapmer and/or comprises phosphothiorate linkages, as described above; and (ii) the gapmer interfering specifically with the expression of SAMMSON is an LNA gapmer and/or comprises phosphothiorate linkages, as described above.

For example, (i) the gapmer interfering specifically with the expression of LINC00518 can comprise or consist of any one of the following sequences: Cbs Cbs Gbs dAs mdCs mdCs dTs dGs dAs dAs dTs dTs dGs Cbs Abs Ab (SEQ ID NO: 5), or Gbs Tbs Abs dGs dAs dGs dGs mdCs dTs dAs dGs dAs dAs Cbs Tbs Gb (SEQ ID NO: 6); and (ii) the gapmer interfering specifically with the expression of SAMMSON can comprise or consist of the following sequence: Gbs Tbs Gbs dTs dGs dAs dAs dmCs dTs dTs dGs Gbs Cbs Tb (SEQ ID NO: 7); wherein A is an adenine, mC is a 5-methylcytosine, G is a guanine, T is a thymine, b is LNA (2',4'-methylene bridged sugar moiety), d is a 2'-deoxyribosyl sugar moiety, and s is a phosphorothioate internucleotide (or internucleoside) linkage.

Other combination of inhibitors of the functional expression of the lncRNAs LINC00518 and SAMMSON can also be envisioned, such as:

- (i) a nucleic acid molecule interfering specifically with the expression of LINC00518, as described above; and (ii) a genome editing system comprising a nuclease engineered to target specifically SAMMSON, as described above; or
- (i) a genome editing system comprising a nuclease engineered to target specifically LINC00518, as described above; and (ii) a nucleic acid molecule interfering specifically with the expression of SAMMSON, as described above; or
- (i) a genome editing system comprising a nuclease engineered to target specifically LINC00518, as described above; and (ii) a genome editing system comprising a nuclease engineered to target specifically SAMMSON, as described above.

It shall be further understood that any one of the (i) inhibitors of functional expression of the long-non coding RNA (lncRNA) LINC00518, and any one of the (ii) inhibitors of functional expression of the long-non coding RNA (lncRNA) SAMMSON, as described herein, can be further combined with an additional therapy, especially one that is suited for melanoma. This includes, without limitation, surgery, radiotherapy, chemotherapy, targeted therapy or immune checkpoint therapy, or any combination thereof. Such therapies are well-known in the art, and therefore need not be detailed herein.

The combination of (i) an inhibitor of functional expression of the long-non coding RNA (lncRNA) LINC00518, and (ii) an inhibitor of functional expression of the long-non coding RNA (lncRNA) SAMMSON, as described herein, may be suitable for administration to a cell, tissue and/or an organ of individuals affected by or at risk of suffering from a melanoma, and may be administered *in vivo, ex vivo* or *in vitro*. Since melanoma affects melanocytes, it is preferred that said cells are melanocytes, and/or that said tissue or organ is the skin.

Whether the combination is administered simultaneously, separately or sequentially, each inhibitor can be delivered as it is to the subject, or be formulated to be compatible with their intended route of administration.

For example, the combination can be formulated in a combined pharmaceutical composition, or in separate pharmaceutical compositions, in a form suitable for parenteral, oral, transdermal or topical administration, such as a liquid suspension, a solid dosage form (granules, pills, capsules or tablets), or a paste or gel.

For the purposes of the invention, a particularly preferred form of administration is parenteral administration, such as subcutaneous, intradermal, intravenous; transdermal administration; or topical administration.

It is within the skill of the person in the art to formulate such pharmaceutical composition(s) in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York). Pharmaceutical compositions according to the invention may notably be formulated to release the combination therapy immediately upon administration or at any predetermined time or time period after administration.

When formulated within a pharmaceutical composition, the combination can be further combined with a pharmaceutically acceptable excipient.

As used herein, the term a *"pharmaceutically acceptable excipient"* means an inactive or inert, and therefore nontoxic, component, as it has no pharmacological action itself, which can be used to improve properties of a composition, such as shelf-life, retention time at the application site, consumer acceptance, etc. It includes, without limitation, surfactants (cationic, anionic, or neutral); surface stabilizers; other enhancers, such as preservatives, wetting or emulsifying agents; solvents; buffers; salt solutions; dispersion medium; isotonic and absorption delaying agents, and the like; that are physiologically compatible.

As explained above, the combination therapy is provided in a therapeutically effective amount so as to treat the melanoma.

In a preferred embodiment, the amount of each inhibitor is a pediatric dose in monotherapy, or a dose that is subtherapeutic in monotherapy (yet, is such that it is therapeutically effective in the combination therapy of the invention). In other words, the amount of each inhibitor is a subtherapeutic dose (yet, is such that it is therapeutically effective in the combination therapy of the invention).

Those of skill in the art will recognize that such parameters are normally worked out during clinical trials.

In another aspect, the present invention relates to (i) an inhibitor of functional expression of the long-non coding RNA (lncRNA) LINC00518, and (ii) an inhibitor of functional expression of the long-non coding RNA (lncRNA) SAMMSON, as described above, as a combined preparation for simultaneous, separate or sequential use in the treatment of a melanoma.

The invention relates to the simultaneous, separate or sequential use of (i) an inhibitor of functional expression of the long-non coding RNA lncRNA) LINC00518 and (ii) an inhibitor of functional expression of the long-non coding RNA (lncRNA) SAMMSON, as described above, for the preparation of a medicament for the treatment of a melanoma.

Further provided is therefore a method for treating a melanoma, in a subject in need thereof, said method comprising the simultaneous, separate or sequential administration, to said subject, of a therapeutically effective amount of (i) an inhibitor of functional expression of the long-non coding RNA (lncRNA) LINC00518 and (ii) an inhibitor of functional expression of the long-non coding RNA (lncRNA) SAMMSON, as described above.

Preferred embodiments, as described above, apply herein *mutatis mutandis.*

In another aspect, the present invention relates to a pharmaceutical composition comprising an inhibitor of functional expression of the long-non coding RNA (lncRNA) LINC00518 and (ii) an inhibitor of functional expression of the long-non coding RNA (lncRNA) SAMMSON, as described above, and optionally (iii) a pharmaceutically acceptable excipient.

Preferred embodiments, as described above, apply herein *mutatis mutandis.*

In another aspect, the present invention pertains to an *in vitro* method for identifying a melanoma tumor suitable for the combined treatment of the invention, said method comprising:

a) determining the respective expression level of the long-non coding RNAs (lncRNAs) LINC00518 and SAMMSON, in a melanoma tumor sample;
b) comparing the expression level determined in step a) with a reference expression level for each of said lncRNA, thereby identifying whether the melanoma tumor is suitable for said treatment.

The term *"expression level",* as applied to a lcnRNA, refers herein to the amount or level of said lncRNA expressed in biological sample, such as a cell, tissue, or organ(s), herein preferably melanocytes or skin. The term *"level"* as used herein refers to an amount (e.g. relative amount or concentration) of a lncRNA that is detectable or measurable in a sample. For example, the level can be a relative amount by comparison to a reference expression level. The act of actually *"determining the expression level"* of a lncRNA in a biological sample refers to the act of actively detecting whether the lcnRNA is expressed in said sample or not, and notably allows to detect whether the expression of the lcnRNA is upregulated, downregulated or substantially unchanged when compared to a reference expression level.

By *"reference expression level"* or *"control expression level",* as applied to a lcnRNA, it is meant a predetermined expression level of said lcnRNA, which can be used as a reference in the method of the invention. For example, a reference expression level can be the expression level of the lncRNA in a biological sample of a healthy subject, or the average or median expression level in a biological sample of a population of healthy subjects.

In a preferred embodiment, step a) is performed by PCR, such as quantitative PCR (qPCR).

In a preferred embodiment, an increased expression level of LINC00518 in step a) and increased expression level of SAMMSON in step a) is indicative of the suitability of said treatment, especially when the reference expression level is one of a healthy subject or population of heathy subjects.

In a further aspect, the present invention is directed to a kit for use in the *in vitro* method for identifying a melanoma tumor suitable for treatment with the inhibitors, said kit comprising:

a) at least one reagent capable of specifically determining the expression level of the long-non coding RNA (lncRNA) LINC00518; and
b) at least one reagent capable of specifically determining the expression level of the long-non coding RNA (lncRNA) SAMMSON;
c) optionally, instructions for performing said method.

As used herein, the term "*instructions*" refers to a publication, a recording, a diagram, or any other medium which can be used to communicate how to perform a method of the invention. Said instructions can, for example, be affixed to a container which contains said kit. Preferably, the instructions for using said kit include the reference expression level for each lncRNA.

The term *"reagent capable of specifically determining the expression level",* as applied to a lcnRNA, designates a reagent or a set of reagents which specifically recognizes said lncRNA, and allows for the quantification of the expression level thereof. These reagents can be for example nucleotide probes (also known as primers). In the context of the present invention, such reagent is said to be *"specific"* for the lncRNA or *"recognizes specifically"* the lncRNA if it 1) exhibits a threshold level of hydridization, and/or 2) does not significantly cross-react with other targets. The hydridization capacity and cross-reactivity of a reagent can be easily determined by one skilled in the art, and thus need to be further detailed herein.

The present invention will be better understood in the light of the following detailed experiments. Nevertheless, the skilled artisan will appreciate that the present examples are not limitative and that various modifications, substitutions, omissions, and changes may be made without departing from the scope of the invention.

## EXAMPLES

## EXAMPLE 1

1. MATERIALS AND METHODS

### 1.1. Gapmers

Gapmers were synthetized and provided by Qiagen (Antisense LNA® GapmeRs).

**Table 3. Gapmers for individual and combinatorial knockdown**

| Name | Sequence (5' to 3') (SEQ ID NO:) | Gapmer type |
|---|---|---|
| Gap-CTR | AACACGTCTATACGC (SEQ ID NO: 1) | LNA (locked nucleic acid) |
| Gap-LINC00518#1 | CCGACCTGAATTGCAA (SEQ ID NO: 2), such as SEQ ID NO: 5 | LNA (locked nucleic acid) |
| Gap-SAMMSON | GTGTGAACTTGGCT (SEQ ID NO: 4), such as SEQ ID NO: 7 | LNA (locked nucleic acid) |

### 1.2. Cell culture

Melanoma cell lines 501mel and Sk-mel-25R were grown in RPMI 1640 medium supplemented with 10% Fetal Calf Serum (FCS) and gentamycin; while melanoma cell lines MM011 and MM099 were grown in HAM-F10 medium supplemented with 10% FCS, 5.2 mM glutamax, 25 mM Hepes and penicillin/streptomycin (7.5 ug/ml). To assess cell growth and viability, cells were stained with Trypan Blue (Invitrogen).

### 1.3 Gapmer transfections

For knockdown, melanoma cells were transfected using Lipofectamine RNAiMAX (Invitrogen) with suboptimal doses of gapmer and harvested 72 hours later for the flow cytometry analysis or 10 days later for the area occupation assay.

**Table 4. Doses of gapmers for individual and combinatorial knockdown**

| Transfection with ASO-LNA Gapmers | Doses of ASO-LNA Gapmers |
|---|---|
| Gap-CTR | 20 nM |
| Gap-CTR+ Gap-LINC00518 | 5 nM + 15 nM |
| Gap-CTR+ Gap-SAMMSON | 15 nM + 5 nM |
| Gap-LINC00518+ Gap-SAMMSON | 15 nM + 5 nm |

### 1.4. Proliferation and survival analyses by flow cytometry

To assess cell proliferation after gapmer-mediated knockdown, cells were stained with Cell Trace Violet (Invitrogen) on the day of transfection harvested after 72 hours and stained with Annexin-V (Biolegend) and TOPRO-3 (Invitrogen) according to the manufacturer's instructions.

To assess cell survival after gapmer-mediated knockdown, cells were stained with the active caspase 3 kit (BD Biosciences) according to the manufacturer's instructions (activated caspase 3 is a marker of cell apoptosis).

Cells were analysed on a LSRII Fortessa (BD Biosciences) and data were analysed with the Flowjo software (Tree Star).

*1.5. Proliferation analysis by the area occupation assay*

When Colony forming ability was assessed, melanoma cells were seeded at low density (500 cells/9.6cm2), transfected with the gapmers, cultured for 10 days and finally fixed in formalin and stained with 0.05% Crystal Violet solution (Sigma Aldrich).

Whole cells were scanned and the pictures were analyzed with ImageJ to calculate the percentage of area occupied.

2. RESULTS

Since the long-non coding RNAs LINC00518 and SAMMSON are expressed in all melanoma cell states, it was investigated whether these could act cooperatively with one another.

To do so, suboptimal concentrations of gapmers for each lncRNA were used either alone or in combination, using a control (CTR) gapmer to ensure a constant amount of gapmer in each condition, in various melanoma cell lines, including 501Mel **(Figure 1),** MM011 **(Figure** 2), MM047 **(Figure 3)** or Sk-mel-25R **(Figure 4).** The proliferation and apoptosis of each cell line were assessed following treatment with the gapmer(s).

Compared to individual knockdown of SAMMSON or LINC00518, combinatorial knockdown led to a more potent reduction in proliferation of 501Mel and MM011 cells **(Figures 1A and 2A)** as well as a strong increase in apoptotic cells (**Figures 1B and 2B).**

Similarly, combinatorial SAMMSON and LINC00518 knockdown in undifferentiated MM047 and Sk-Mel-25R cells also cooperatively induced a potent increase in apoptotic cells **(Figures 3B and 4B).**

The synergistic effect of combinatorial LINC00518-SAMMSON silencing was also evidenced after 10 days of culture, by which time about 90% of MM011 cells were eliminated **(Figure 2C),** and essentially all the MM047 cells were eliminated **(Figure 3C).**

LINC00518 and SAMMSON therefore act cooperatively to promote melanoma cell proliferation and survival, and can be inhibited to treat melanoma.

3. CONCLUSION

The combination therapy according to the invention, notably at low doses, provides unexpected superior benefits than either treatment alone, since it can synergistically (i) reduce the proliferation of melanoma cells and (ii) increase the apoptosis of these cells.

While this combination therapy is suited to all melanoma states, it is of particular benefit for undifferentiated cell states that play an essential role in residual/resistant disease and relapse.

**EXAMPLE 2**

A similar experiment to EXAMPLE 1 is conducted with a different type of lncRNA inhibitors. This time, individual knockdown of SAMMSON or LINC00518, as well as combinatorial knockdown thereof, are performed in a melanoma cell line using RNAi nucleic acids targeting each lncRNA. A control (CTR) RNAi nucleic acid is also used to ensure a constant amount of RNAi nucleic acids in each condition.

To do so, melanoma cells are transfected with suboptimal concentrations of shRNA or siRNA for each lncRNA, used either alone or in combination, using a control (CTR) shRNA to ensure a constant amount of sh/siRNA in each condition.

**Table 5. RNAi nucleic acids for individual and combinatorial knockdown**

| Name | Sequence (5' to 3') (SEQ ID NO:) |
|---|---|
| i-CTR | ATTACGTCTGTCATGAACCTC (SEQ ID NO: 8) |
| i-LINC00518 | TTCACTAAGGTTCCATCTAGC (SEQ ID NO: 9) |
| i-SAMMSON | GUCGCUAGACAUUUGAGGA[dA][dA]] (SEQ ID NO: 10) |

**EXAMPLE 3**

A similar experiment to EXAMPLE 1 is conducted with a different type of lncRNA inhibitors. This time, individual knockdown of SAMMSON or LINC00518, as well as combinatorial knockdown, are performed in a melanoma cell line using vectors encoding the CRISPR/dCAS9-KAP1 fusion protein with or without (CTR) sgRNA targeting it to the LINC518 or SAMMSON promoter region.

To do so, melanoma cells are co-transfected using Fugene 6 (Promega) with a plasmid expressing dead Cas9 protein fused to the Kruppel-associated box (KRAB) domain-containing KAP1 (dCas9-KAP1) and the red fluorescent protein mScarlet (pX-dCas9-KRAB-Scarlet), together with a plasmid expressing GFP and three single guide RNAs targeting the transcription start site of LINC00518 or SAMMSON or a control plasmid expressing GFP only (pCMV-GFP). Double Scarlet-GFP positive cells are sorted 24 hours after co-transfection, stained with Cell Trace Violet and kept in culture for additional 96 hours. Cells are harvested to prepare total RNA and stained with AnnexinV-APC (Biolegend). Cells are analysed on a LSRII Fortessa (BD Biosciences) and data were analysed with Flowjo software (Tree Star).

**Table 6. Single guide RNAs for individual and combinatorial knockdown**

| Name | Sequence (5' to 3') (SEQ ID NO:) |
|---|---|
| sg-LINC00518#1 | CCGAGAGAGAGGACTCTCTG (SEQ ID NO: 11) |
| sg-LINC00518#2 | TGTTACTAATGAGACCGCTG (SEQ ID NO: 12) |
| sg-LINC00518#3 | ACTGATACACACTATAAACG (SEQ ID NO: 13) |

**EXAMPLE 4**

A similar experiment to EXAMPLE 1 is conducted in a melanoma cell line for knockdown of SAMMSON and LINC00518, by using:

- a GapmeR for one lncRNA, combined with a RNAi nucleic acid for the other lncRNA;
- a GapmeR for one lncRNA, combined with a vector encoding the CRISPR/dCAS9-KAP1 fusion protein with sgRNA targeting the promoter region of the other lncRNA;
- a RNAi nucleic acid for one lncRNA, combined with vector encoding the CRISPR/dCAS9-KAP1 fusion protein with sgRNA targeting the promoter region of the other lncRNA.

**Claims**

1. (i) An inhibitor of functional expression of the long-non coding RNA (lncRNA) LINC00518 for use in combination with (ii) an inhibitor of functional expression of the long-non coding RNA (lncRNA) SAMMSON, in the treatment of a melanoma.

2. The inhibitors (i) and (ii) for use according to claim 1, wherein the inhibitors increase the apoptosis and/or decrease the proliferation of melanoma cells.

3. The inhibitors (i) and (ii) for use according to claim 2, wherein the inhibitors increase apoptosis and/or decrease the proliferation of melanoma cells, independent of BRAF and/or NRAS status.

4. The inhibitors (i) and (ii) for use according to any one of claims 1 to 3, wherein the melanoma is an advanced melanoma or a metastatic melanoma.

5. The inhibitors (i) and (ii) for use according to any one of the preceding claims, wherein the melanoma is a resistant melanoma, in particular a melanoma resistant to chemotherapy, targeted therapy, and/or immune checkpoint inhibitors.

6. The inhibitors (i) and (ii) for use according to any one of the preceding claims, wherein each inhibitor is either a nucleic acid molecule interfering specifically with the expression of the long-non coding RNA, or is a genome editing system comprising a nuclease engineered to target specifically the long-non coding RNA.

7. The inhibitors (i) and (ii) for use according to claim 6, wherein the nucleic acid molecule interfering specifically with the expression of the long-non coding RNA is an antisense nucleic acid, an RNAi nucleic acid, or a ribozyme, preferably is an antisense nucleic acid.

8. The inhibitors (i) and (ii) for use according to claim 7, wherein the antisense nucleic interfering specifically with the expression of the long-non coding RNA induces a RNAse H mediated degradation.

9. The inhibitors (i) and (ii) for use according to claim 7 or 8, wherein the antisense nucleic acid interfering specifically with the expression of the long-non coding RNA is a gapmer.

10. The inhibitors (i) and (ii) for use according to any one of claims 7 to 9, wherein the antisense is an LNA antisense and/or comprises phosphothiorate linkages, preferably the gapmer is an LNA gapmer and/or comprises phosphothiorate linkages.

11. The inhibitors (i) and (ii) for use according to claim 10, wherein the genome editing system comprising a nuclease engineered to target specifically the long-non coding RNA is the CRISPR/Cas system, the Zinc-finger nuclease (ZFN) system, the TALEN system, or the meganuclease system, preferably is the CRISPR/Cas system such as the CRISPR/Cas9 system.

12. (i) An inhibitor of functional expression of the long-non coding RNA (lncRNA) LINC00518 and (ii) an inhibitor of functional expression of the long-non coding RNA (lncRNA) SAMMSON, as defined in any one of the preceding claims, as a combined preparation for simultaneous, separate or sequential use in the treatment of a melanoma.

13. A pharmaceutical composition comprising (i) an inhibitor of functional expression of the long-non coding RNA (lncRNA) LINC00518 and (ii) an inhibitor of functional expression of the long-non coding RNA (lncRNA) SAMMSON, as defined in any one of the preceding claims, and optionally (iii) a pharmaceutically acceptable excipient.

14. An *in vitro* method for identifying a melanoma tumor suitable for treatment with the inhibitors as defined in any one of the above claims, said method comprising:

    a) determining the respective expression level of the long-non coding RNAs (lncRNAs) LINC00518 and SAMMSON, in a melanoma tumor sample;
    b) comparing the expression level determined in step a) with a reference expression level for each of said lncRNA, thereby identifying whether the melanoma tumor is suitable for said treatment.

15. A kit for use in the method of claim 14, comprising:

    a) at least one reagent capable of specifically determining the expression level of the long-non coding RNA (lncRNA) LINC00518; and
    b) at least one reagent capable of specifically determining the expression level of the long-non coding RNA (lncRNA) SAMMSON;
    c) optionally, instructions for performing said method.

**501meI**

A

B

**FIGURE 1**

## MM011

A

B

C

**FIGURE 2**

## MM047

FIGURE 3

SKMEL25R

A                                                    B

FIGURE 4

Europäisches
Patentamt

European
Patent Office

Office européen
des brevets

# EUROPEAN SEARCH REPORT

**Application Number**

EP 22 30 5950

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2015/024986 A1 (VIB VZW [BE]; UNIV LEUVEN KATH [BE]; UNIV GENT [BE]) 26 February 2015 (2015-02-26) * the whole document * | 1-15 | INV. C12N15/113 A61K31/7125 A61K31/713 A61K31/712 |
| A,D | WO 2021/152005 A1 (UNIV STRASBOURG [FR]; CENTRE NAT RECH SCIENT [FR] ET AL.) 5 August 2021 (2021-08-05) * the whole document * | 1-15 | ADD. A61P35/00 |
| A | WO 2020/239685 A1 (UNIV GENT [BE]) 3 December 2020 (2020-12-03) * the whole document * | 1-15 | |
| A | CN 110 898 213 A (UNIV XI AN JIAOTONG) 24 March 2020 (2020-03-24) * the whole document * | 1-15 | |
| A | WOZNIAK MICHAL ET AL: "The Functional Role of Long Non-Coding RNAs in Melanoma", CANCERS, vol. 13, no. 19, 4848, 2021, pages 1-30, XP055972159, DOI: 10.3390/cancers13194848 Retrieved from the Internet: URL:https://www.ncbi.nlm.nih.gov/pmc/articles/PMC8508152/pdf/cancers-13-04848.pdf> * the whole document * | 1-15 | TECHNICAL FIELDS SEARCHED (IPC) C12N A61K |

The present search report has been drawn up for all claims

| Place of search | Date of completion of the search | Examiner |
|---|---|---|
| The Hague | 29 November 2022 | Andres, Serge |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 30 5950

This annex lists the patent family members relating to the patent documents cited in the above-mentioned European search report.
The members are as contained in the European Patent Office EDP file on
The European Patent Office is in no way liable for these particulars which are merely given for the purpose of information.

29-11-2022

| Patent document cited in search report | | Publication date | Patent family member(s) | | Publication date |
|---|---|---|---|---|---|
| WO 2015024986 | A1 | 26-02-2015 | AU | 2014310564 A1 | 10-03-2016 |
| | | | CA | 2921778 A1 | 26-02-2015 |
| | | | CN | 105705640 A | 22-06-2016 |
| | | | DK | 3036326 T3 | 08-01-2018 |
| | | | EP | 3036326 A1 | 29-06-2016 |
| | | | NO | 3036326 T3 | 03-03-2018 |
| | | | US | RE48801 E | 02-11-2021 |
| | | | US | 2016271163 A1 | 22-09-2016 |
| | | | WO | 2015024986 A1 | 26-02-2015 |
| WO 2021152005 | A1 | 05-08-2021 | AU | 2021213317 A1 | 30-06-2022 |
| | | | CA | 3161513 A1 | 05-08-2021 |
| | | | EP | 4097234 A1 | 07-12-2022 |
| | | | WO | 2021152005 A1 | 05-08-2021 |
| WO 2020239685 | A1 | 03-12-2020 | EP | 3976055 A1 | 06-04-2022 |
| | | | US | 2022218734 A1 | 14-07-2022 |
| | | | WO | 2020239685 A1 | 03-12-2020 |
| CN 110898213 | A | 24-03-2020 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015024986 A
- WO 2021152005 A

- WO 9932619 A

**Non-patent literature cited in the description**

- **LEUCCI et al.** *Nature,* 2016, vol. 531, 518-22
- **JOHNSON et al.** *J. Clin. Oncol.,* 2009, vol. 21 (7), 1404-1411
- **ECKSTEIN F.** *Antisense and Nucleic Acid Drug Development,* 2000, vol. 10 (2), 1117-221
- **WATTS et al.** *J Pathol.,* 2012, vol. 226 (2), 365-379
- **SETH et al.** *J Clin Invest.,* 2019, vol. 129 (3), 915-925
- **LEE et al.** *J Cardiovasc Transl Res.,* 2013, vol. 6 (6), 969-80
- **DEVOS et al.** *Neurotherapeutics,* 2013, vol. 10 (3), 486-972013
- **SOHAIL et al.** Gene Silencing by RNA Interference: Technology and Application. 2004
- **WANG et al.** *Pharm Res,* 2011, vol. 28, 2983-2995

- **BERNSTEIN et al.** *Nature,* 2001, vol. 409 (6818), 363-6
- **ZAMORE et al.** *Cell,* 2000, vol. 101 (1), 25-33
- **ELBASHIR et al.** *Genes Dev.,* 2001, vol. 15 (2), 188-200
- **ELBASHIR et al.** *EMBO J.,* 2001, vol. 20 (23), 6877-88
- RNA Towards Medicine. **FANNING ; SYMONDS.** Handbook of Experimental Pharmacology. Springer, 2006, 289-303
- **MALI et al.** *Nature Methods,* 2013, vol. 10 (10), 957-63
- Remington: The Science and Practice of Pharmacy. Lippincott Williams & Wilkins, 2000
- Encyclopedia of Pharmaceutical Technology. Marcel Dekker, 1988